(19) Europäisches Patentamt
European Patent Office
Office européen des brevets

(11) Numéro de publication : **0 515 257 A1**

(12) # DEMANDE DE BREVET EUROPEEN

(21) Numéro de dépôt : **92401349.3**

(22) Date de dépôt : **15.05.92**

(51) Int. Cl.$^5$ : **C07D 237/16,** C07D 237/14, A61K 31/50

(30) Priorité : **22.05.91 FR 9106148**

(43) Date de publication de la demande : **25.11.92 Bulletin 92/48**

(84) Etats contractants désignés : **AT BE CH DE DK ES FR GB GR IT LI LU MC NL PT SE**

(71) Demandeur : **PIERRE FABRE MEDICAMENT**
**45, Place Abel Gance**
**F-92100 Boulogne (FR)**

(72) Inventeur : **Autin, Jean-Marie**
**Péri-Albo, Viviers Les Montagnes**
**F-81290 Labruguière (FR)**
Inventeur : **Bigg, Dennis**
**122, Avenue de Lavaur**
**F-81100 Castres (FR)**
Inventeur : **Patoiseau, Jean-François**
**7,rue Jules Ferry**
**F-81100 Castres (FR)**

(74) Mandataire : **Doat, Jean Pierre**
**17, Avenue Jean Moulin**
**F-81106 Castres Cedex (FR)**

(54) **Dérivés de phényl-1 dihydro-1,4 hydroxy-3 oxo-4 pyridazines leur préparation et leur application en thérapeutique.**

(57) La présente invention se rapporte à des dérivés de phényl-1 dihydro-1,4 hydroxy-3 oxo-4 pyridazines répondant à la formule générale I :

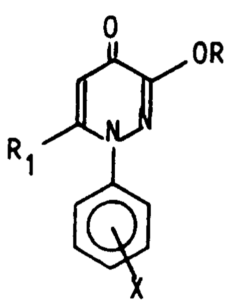

I

Elle concerne également les compositions pharmaceutiques comprenant à titre de principe actif au moins un de ces composés de formule générale I.

EP 0 515 257 A1

La présente invention, réalisée au Centre de Recherche PIERRE FABRE MEDICAMENT, a pour objet de nouveaux composés chimiques, leur procédé de préparation et leur application en tant que médicaments.

Des phényl-1 dihydro-1,4 oxo-4 pyridazines diversement substitués en 3 sont décrits dans la littérature. Parmi ces composés, les acides carboxyliques possèdent une activité sur la régulation de croissance des végétaux et sont utilisés comme gamétocides (demande de brevet FR-A-2,383,605, Romm and Haas Company). D'autres acides, diversement substitués sur l'aromatique, et leurs esters, présentent une activité stimulante du système nerveux central (brevets US 2,792,396 et 2,835,671, Ciba pharmaceutical Products). Des composés aminés en 3, présentant une activité sur le système nerveux central ont été décrits par la demanderesse (demande de brevet FR-A-2.648,135, Pierre Fabre Médicament). En outre, la phényl-1 dihydro-1,4 hydroxy-3 oxo-4 pyridazine a été synthétisée par Stahelin et Coll, (Helv. Chim. Acta 39, p. 1741-1754, 1956) qui ne mentionnent aucune activité pharmacologique.

Les nouveau composés chimiques, revendiqués par la demanderesse, répondent à la formule générale I :

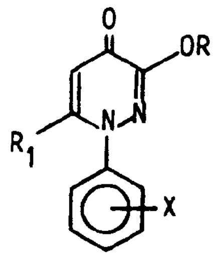

I

dans laquelle :

- X représente un ou plusieurs groupements choisis parmi alcoyle inférieur en $C_{1-4}$, alcoyl $C_{1-4}$ oxy, halogène, trifluorométhyl,
- $R_1$ représente l'hydrogéne ou un groupement alcoyle inférieur en $C_{1-3}$.
- R représente :
  . l'hydrogène;
  . un groupement alcoyle ou alcenyle inférieur en $C_1$-$C_4$;
  . un groupement acyle inférieur en $C_1$-$C_4$;
  . un groupement amino alcoyl II

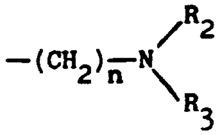

  dans lequel n est un entier compris entre 2 et 4 inclus, et $R_2$, $R_3$ identiques ou différents représentant l'hydrogène ou un groupement alcoyle inférieur $C_1$-$C_4$;
  . un groupement phényl alcoyl $C_1$-$C_4$, le noyau aromatique pouvant être substitué par des groupements tels qu'alcoyle $C_1$-$C_3$, alcoyl $C_1$-$C_3$ oxy, halogène, trifluorométhyl;
  . un groupement aryl méthyl, le groupement aryl étant choisi parmi 2-pyridyl, 3-pyridyl, 4 -pyridyl, 2-quinolinyl.

L'invention couvre les sels organiques ou minéraux thérapeutiquement acceptables des molécules décrites comportant une fonction salifiable.

La présente invention concerne également la prépartion de dérivés de formule I à partir de phényl-1 amino-3 oxo-4 pyridazines IIIa ou d'aryl-1 chloro-3 oxo-4 pyridazines IIIb, X et $R_1$ ayant la même signification que précédemment.

Ces composés intermédiaires peuvent être obtenus selon les descriptions de la demande de brevet FR-A-2.648.135. Pierre Fabre Médicament.

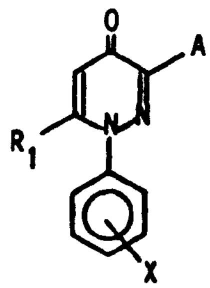

**IIIa** A = $NH_2$

**IIIb** A = Cl

Le procédé, à partir du dérivé IIIa est caractérisé en ce qu'il comprend :

a) une étape qui consiste à transformer l'amine en dérivé hydroxylé par traitement au nitrite de sodium en milieu chlorhydrique ou par un nitrite organique tel que le tertiobutyle nitrite dans un solvant tel qu'un mélange eau-diméthyl formamide.

b) une étape 0-alcoylation par un dérivé RX, R ayant la même signification que précédemment et X représentant un groupe labile tel que chlore, brome ou iode, ou par un dialcoyle sulfate, un mésylate ou un tosylate. Cette réaction peut être effectuée en présence d'une base telle que, à titre d'exemple, NaH ou NaOH, dans un solvant approprié tel que DMF, DMSO, THF, ou diméthyl acétamide.

La réaction peut être avantageusement conduite en milieu biphasique tel que THF-soude en présence d'un catalyseur de transfert de phase tel que le bromure de tétrabutyl ammonium.

Dans le cas où le radical R comporte un groupement fonctionnel tels qu'une amine primaire, le fonction pourra être provisoirement bloquée sous la forme de phtalimide et le produit désiré obtenu selon les méthodes classiques de déprotection telles que le traitement à l'hydrazine, à l'héthanolamine ou à l'éthylène diamine.

Le procédé, à partir du dérivé chloré IIIb est caractérisé en ce qu'il consiste à faire réagir un alcool ROH en présence d'une base, telle que, à titre d'exemple NaH ou KOH dans un solvant approprié tel que DMF, DMSO ou THF.

Les exemples ci-après illustrent la présente invention.

Les analyses centésimales ainsi que les spectres IR et RMN confirment la structure des composés obtenus.

## Exemple 1

**m.trifluoromethyl phenyl-1 dihydro-1,4 hydroxy-3 oxo-4 méthyl-6 pyridazine 1**

Une suspension de m.trifluorométhyl phenyl-1 dihydro-1,4 amino-3 oxo-4 méthyl-6 pyridazine (21 g, 0,078 mole) est glacée dans 400 ml d'eau. Après addition d'acide chlorhydrique 12N (14,5 ml), on ajoute goutte à goutte le nitrite de sodium (5,38 g) dans l'eau (50 ml). Après agitation du mélange 1 heure à 0°C, 2 heures à 20°C puis 10 mn à 50°C, on refroidit, filtre sur fritté et lave à l'eau. Le précipité est repris par 1200 ml d'acétate d'éthyle tiède. Après séchage sur sulfate de sodium, filtration et évaporation sous vide, le produit cristallise après reprise à l'éther éthylique. On obtient, après recristallisation dans un mélange dioxane-éther éthylique 75-25, le composé 1 (18,75 g ; Rdt = 90 %).

F = 242°C

CCM : Rf = 0,21 (chloroforme-méthanol 85-15).

## Exemple 2

**m.trifluoromethyl phenyl-1 dihydro-1,4 méthoxy-3 oxo-4 méthyl-6 pyridazine 2**

A une solution du composé 1 (5,31 g) dans le THF, on ajoute le bromure de tétrabutylammoniun (635 mg), la soude 6N (13 ml) et le sulfate de méthyle (5,59 ml). Aprés 1 H 30 d'agitation à 25°C, la solution est évaporée sous vide, reprise à l'eau et extraite à l'acétate d'éthyle. On obtient, apis lavage à l'eau, séchage, filtration et évaporation sous vide, un résidu qui est repris à l'éther et recristallise d'un mélange dioxane-hexane 60-40 pour donner le composé 2

(3,97 g ; Rdt = 71 %),

F = 172°C

CCM : Rf = 0,39 ($CHCl_3$-MeOH 85-15).

**Exemple 3**

**m.trifluoromethyl phenyl-1 dihydro-1,4 acétoxy-3 oxo-4 méthyl-6 pyridazine 3**

Une suspension du composé 1 (2,36 g) dans l'anhydride acétique est chauffée pendant 6 heures à 50°C, La solution, refroidie, est concentré sous vide. On obtient, après addition d'éther éthylique, filtration, lavé à l'éther et séchage, le composé 3 (1,91 g ; Rdt = 70 %).
F = 153-154°C
CCM : Rf = 0,77 (méthanol-chloroforme 15-85).

**Exemple 4**

**m.trifluoromethyl phenyl-1 dihydro-1,4 β amino éthoxy-3 oxo-4 méthyl-6 pyridazine 7**

A une suspension d'hydrure de sodium (0,084 g) dans le diméthylacétamide (14 ml), on ajoute le composé 1 (1,35 g). On introduit ensuite le bromo-1 phtalimido-2 éthane (2,54 g) et agite 3 H à 120°C. Après évaporation sous vide du diméthylacétamide, on dilue à l'eau et extrait à l'acétate d'éthyle. La solution organique, lavée à la soude 2N puis à l'eau est séchée sur sulfate de sodium, filtrée et concentrée. On obtient après glaçage, 1,26 g de cristaux blancs (dérivé phtalimido intermédiaire).
Ce composé (1,09 g) est repris dans l'éthylène diamine (7 ml) et agité 4 H à température ambiante. Après évaporation sous vide de l'éthylène diamine, le résidu est repris par l'eau et extrait à l'acétate d'éthyle.
Apis séchage sur sulfate de sodium, filtration et concentration à sec, on obtient par reprise à l'éther éthylique le composé 7 (0,67 g ; Rdt global = 49 %).
F = 160°C
CCM : Rf = 0,70 (méthanol-chloroforme 15-85 ).

**Exemple 5**

**m.trifluoromethyl phenyl-1 dihydro-1,4 oxo-4 (quinolyl-2 méthoxy)-3 méthyl-6 pyridazine 11**

Une solution d'hydroxy-2 méthyl quinoléine ( 1, 34 g) dans le THF (20 ml), est portée à 0°C. On ajoute par petites portions l'hydrure de sodium (0,34 g) puis après 30 mn on introduit la m.trifluorométhyl phényl-1 dihydro-1,4 chloro-3 oxo-4 méthyl-6 pyridazine (2,08 g). Apis 30 mn à 0°C et 4 heures à température ambiante, le mélange fractionnel est concentré à sec, repris à l'eau et extrait à l'acétate d'éthyle. La phase organique, lavée à l'eau, séchée sur sulfate de sodium est filtrée et concentrée à sec sous vide.
On obtient après purification par flash chromatographie (éluant $CHCl_3$-MeOH 95-5), le composé 11 (1,35 g ; Rdt = 39 %).
F = 189°C
CCM : Rf = 0,4 ($CHCl_3$-MeOH 95-5).

Le tableau ci-après résume les principaux produits synthétisés qui illustrent l'invention sans en limiter la portée.

| N° | X | $R_1$ | R | F°C |
|---|---|---|---|---|
| 1 | m.$CF_3$ | $CH_3$ | H | 242 |
| 2 | m.$CF_3$ | $CH_3$ | $CH_3$ | 172 |
| 3 | m.$CF_3$ | $CH_3$ | $COCH_3$ | 153-154 |
| 4 | m.$CF_3$ | $CH_3$ | $CH_2CH_3$ | 158-159 |
| 5 | m.$CF_3$ | $CH_3$ | $CH_2-CH=CH_2$ | 138 |
| 6 | m.$CF_3$ | $CH_3$ | $CH_2-\phi$ | 134 |
| 7 | m.$CF_3$ | $CH_3$ | $CH_2CH_2NH_2$ | 160 |
| 8 | m.$CF_3$ | $CH_3$ | $CH_2CH_2N(CH_3)_2$ | 138 (fumarate) |
| 9 | m.$CF_3$ | H | $CH_3$ | 154 |
| 10 | m.$CF_3$ | H | $CH_2-\phi$ | 123 |
| 11 | m.$CF_3$ | $CH_3$ | $-CH_2$ 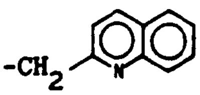 | 189 |
| 12 | m.Cl | $CH_3$ | $CH_3$ | 174 |
| 13 | m.$CH_3$ | $CH_3$ | $CH_3$ | 175 |
| 14 | m.F | $CH_3$ | $-CH_2$ 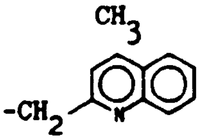 | 177 |
| 15 | p.F | $CH_3$ | $-CH_2$ 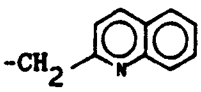 | 190 |
| 16 | 3-4 diméthoxy | H | $CH_3$ | 107-108 |
| 17 | 3-4-5 triméthoxy | H | $CH_3$ | 156 |
| 18 | 3-4-5 triméthoxy | $CH_3$ | $CH_3$ | 219 |

## Expérimentations

Divers essais toxicologiques et pharmacologiques ont été effectués sur les composés, objets de la présente invention.

## A - Toxicologie

Les composés de l'invention ont été soumis à des contrôles de toxicologie.
Celle-ci a été déterminée par la dose létale 50 % (DL50), Elle a été recherchée sur des lots de 10 souris par voie orale et calculée selon la méthode de Thomson et Weil (Biometrics, 1958, 8, 249).
Les DL50 des composés testés sont supérieures à 300 mg/kg par voie orale.

**B - Propriétés pharmacologiques**

Les expérimentations pharmacologiques ont permis de mettre en évidence de remarquables propriétés sur le système nerveux central d'une part, et en tant qu'inhibiteurs de lipoxygénase, d'autre part.

**1 - Activité anxiolytique**

L'activité anxiolytique des composés de la présente invention a été mise en évidence sur le test de Vogel (Thiebot M.H. et al., Eur. J. Pharma. 88, p.111-116, 1987) et sur le test du labyrinthe en X (elevated plus maze) (Pellow S. et al., J. Neurosci. Methods 1985, 14, 149-167).
Ci-après, sont reportés, à titre d'exemple, les résultats obtenus sur certains produits de la présente invention,

Les résultats sont exprimés en pourcentage d'augmentation par rapport aux témoins ; le produit étant administré per os à la dose de 30 mg/kg.

| Produit | Test de Vogel | Test du labyrinthe en X |
|---|---|---|
| 2 | 95 % | 95 % |
| 5 | 48 % | - |
| 6 | 98 % | 18 % |
| 9 | 52 % | 165 % |
| 10 | 38 % | - |

**2 - Activité inhibitrice de lipoxygénase**

Cette activité a été mise en évidence sur le test d'inhibition de la production de leucotriène B4 sur des suspensions de polymorphonucléaires péritonéaux de rat stimulés par l'Ionophore A 23 187.

Ci-après sont reportés, à titre d'exemple, les résultats obtenus sur certains produits de l'invention.

| Produits | $CI_{50}$ (µMol) |
|---|---|
| 6 | 1.25 |
| 10 | 25 |
| 11 | 1.0 |

**3 - Applications thérapeutiques**

Compte tenu de leurs propriétés pharmacologiques, les composés de la présente invention peuvent être utilisés en thérapeutique humaine dans le traitement de diverses maladies et plus particulièrement l'anxiété, l'asthme, l'inflammation rhumatismale, les maladies inflammatoires digestives et du côlon, le psoriasis.

Les préparations pharmaceutiques contenant ces principes actifs peuvent être mises en forme pour l'administration par voie orale, injectable, rectale ou parentérale, par exemple capsules, comprimés, gélules, solutés contenant les excipients appropriés.

Il est également possible d'y associer d'autres principes actifs pharmaceutiquement et thérapeutiquement acceptables.

## Revendications

**1 -** Composés phényl-1 dihydro-1,4 oxo-4 pyridazines correspondant à la formule générale I

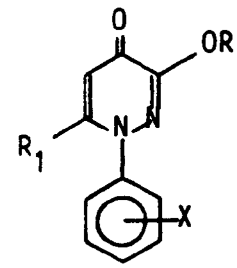

I

dans laquelle :

- X représente un ou plusieurs groupements choisis parmi alcoyle inférieur en $C_{1-4}$, alcoyl $C_{1-4}$ oxy, halogéne, trifluorométhyl.
- $R_1$ représente l'hydrogène ou un groupement alcoyle inférieur en $C_{1-3}$.
- R représente :
  . l'hydrogène;
  . un groupement alcoyle ou alcenyle inférieur en $C_1$-$C_4$;
  . un groupement acyle inférieur en $C_1$ -$C_4$;
  . un groupement amino alcoyl II

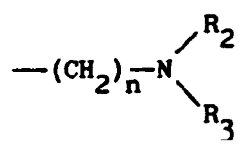

dans lequel n est un entier compris entre 2 et 4 inclus, et $R_2$, $R_3$ identiques ou différents représentant l'hydrogène ou un groupement alcoyle inférieur $C_1$-$C_4$;
  . un groupement phényl alcoyl $C_1$-$C_4$, le noyau aromatique pouvant être substitué par des groupements tels qu'alcoyle $C_1$-$C_3$, alcoyl $C_1$-$C_3$ oxy, halogène, trifluorométhyl;
  . un groupement aryl méthyl, le groupement aryl étant choisi parmi 2-pyridyl, 3-pyridyl, 4-pyridyl, 2-quinolinyl.

**2 -** Composée de formule générale I selon la revendication 1 caractérisés par le fait qu'ils sont choisis parmi :

- metatrifluoromethyl phenyl-1 dihydro-1,4 hydroxy-3 oxo-4 methyl-6 pyridazine
- metatrifluoromethyl phenyl-1 dihydro-1,4 methoxy-3 oxo-4 methyl-6 pyridazine
- metatrifluoromethyl phenyl-1 dihydro-1,4 acetoxy-3 oxo-4 methyl-6 pyridazine
- metatrifluoromethyl phenyl-1 dihydro-1,4 ethoxy-3 oxo-4 methyl-6 pyridazine
- metatrifluoromethyl phenyl-1 dihydro-1,4 allyloxy-3 oxo-4 methyl-6 pyridazine
- metatrifluoromethyl phenyl-1 dihydro-1,4 benzyloxy-3 oxo-4 methyl-6 pyridazine
- metatrifluoromethyl phenyl-1 dihydro-1,4 β amino ethoxy-3 oxo-4 methyl-6 pyridazine
- metatrifluoromethyl phenyl-1 dihydro-1,4 β dimethyl amino ethoxy-3 oxo-4 methyl-6 pyridazine
- metatrifluoromethyl phenyl-1 dihydro-1,4 methoxy-3 oxo-4 pyridazine
- metatrifluoromethyl phenyl-1 dihydro-1,4 benzyloxy-3 oxo-4 pyridazine
- metatrifluoromethyl phenyl-1 dihydro-1,4 (quinolyl-2 methoxy)-3 oxo-4 methyl-6 pyridazine
- metachlorophenyl-1 dihydro-1,4 methoxy-3 oxo-4 methyl-6 pyridazine
- metatolyl-1 dihydro-1,4 methoxy-3 oxo-4 methyl-6 pyridazine
- metafluorophenyl-1 dihydro-1,4 (quinolyl-2 methoxy)-3 oxo-4 methyl-6 pyridazine
- parafluorophenyl-1 dihydro-1,4 (quinolyl-2 methoxy)-3 oxo-4 methyl-6 pyridazine
- (dimethoxy-3-4)phenyl-1 dihydro-1,4 methoxy-3 oxo-4 pyridazine
- (trimethoxy-3-4-5)phenyl-1 dihydro-1,4 methoxy-3 oxo-4 pyridazine

- (trimethoxy-3-4-5)phenyl-1 dihydro-1,4 methoxy-3 oxo-4 methyl-6 pyridazine

**3 -** Procédé de préparation de composés selon les revendications 1 ou 2 caractérisé en ce que l'on traite une amino-3 pyridazine IIIa par le nitrite de sodium en milieu chlorhydrique ou un nitrite organique tel que le nitrite de tertiobutyle dans un solvent ou un mélange de solvant tel que eau-DMF pour obtenir les composés avec R=H

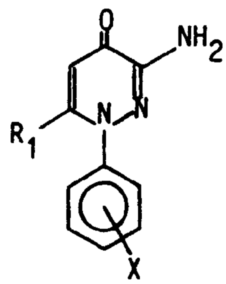

**IIIa**

**4 -** Procédé de préparation de composés selon les revendications 1 ou 2, caractérisé en ce que l'on traite une hydroxy-3 pyridazine obtenue selon la revendication 3 par un composé RX, R ayant la même signification que précédemment et X représentant un halogène tel que iode, chlore ou brome ou par un sulfate de dialcoyle $(R)_2SO_4$, un mésylate ou un tolysate.

**5 -** Procédé de préparation de composés selon la revendication 4 caractérisé en ce que la réaction est effectuée en présence d'une base telle que NaH ou NaOH.

**6 -** Procédé de préparation de composés selon, les revendications 4 et 5 caractérisé en ce que la réaction peut-être effectuée dans un solvant tel que DMF, DMSO, dimethyl acetamide ou tetrahydrofurane.

**7 -** Procédé de préparation de composés selon la revendication 4 caractérisé en ce que la réaction peut-être effectuée dans un milieu biphasique tel que soude-THF en présence d'un catalyseur de transfert de phase.

**8 -** Procédé de préparation de composés selon les revendications 1 ou 2 caractérisé en ce que l'on traite une chloro-3 pyridazine IIIb par un alcool ROH, X, $R_1$ et R ayant les mêmes significations que précédemment.

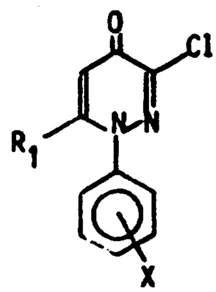

**IIIb**

**9 -** Procédé de préparation de composés selon la revendication 8 caractérisé en ce que la réaction est effectué en présence d'une base telle que NaH ou KOH dans un solvant tel que DMF, DMSO, THF.

**10 -** A titre de médicaments nouveaux, utiles par exemple dans le traitement de l'anxiété, de l'asthme, de l'inflammation rhumatismale, des maladies inflammatoires digestive ou du côlon, du psoriasis, les composés définis selon l'une des revendications 1 et 2.

**11 -** Compositions pharmaceutiques caractérisées en ce qu'elles contiennent comme principe actif un composé défini selon l'une des revendications 1 ou 2.

**12 -** Compositions pharmaceutiques selon la revendication 11 caractérisées en ce qu'elles contiennent en outre un autre principe actif.

Office européen des brevets

# RAPPORT DE RECHERCHE EUROPEENNE

Numero de la demande

EP 92 40 1349

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A<br><br>A,D | EP-A-0 402 227 (PIERRE FABRE MEDICAMENT)<br>* revendications 1,14,16-19 *<br>& FR-A-2 648 135 | 1,3,8, 10-12 | C07D237/16<br>C07D237/14<br>A61K31/50 |
| A<br><br><br><br><br><br><br>A,D | CHEMICAL ABSTRACTS, vol. 51, no. 6, 25 Mars 1957, Columbus, Ohio, US; abstract no. 4380D-4383C, A. STAEHELIN ET AL.: 'Chemotherapeutic studies in the heterocyclic series ...'<br>colonne 4380 ;<br>* colonne 4380h-i *<br>& Helv. Chim. Acta 39, 1741-54 (1956) | 1,3 | |
| A<br><br><br><br><br><br><br><br>A,D | CHEMICAL ABSTRACTS, vol. 58, no. 5, 4 Mars 1963, Columbus, Ohio, US; abstract no. 4540B-E, H.D. STACHEL: 'Ketene derivatives. IV. Acetyl ketene mercaptals'<br>colonne 4540 ;<br>* composé (VIa) *<br>& Arch. Pharm. 295, 224-33 (1962) | 1 | **DOMAINES TECHNIQUES RECHERCHES (Int. Cl.5 )** |
| A,D | FR-A-2 383 605 (ROHM AND HAAS CO.)<br>* page 2, ligne 1 - ligne 20 * | 1 | C07D |

Le présent rapport a été établi pour toutes les revendications

| Lieu de la recherche | Date d'achèvement de la recherche | Examinateur |
|---|---|---|
| BERLIN | 24 AOUT 1992 | CHRISTIAN HASS |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)